# EUROPEAN PATENT SPECIFICATION

(11) **EP 3 954 340 B1**
(45) Date of publication and mention of the grant of the patent: **28.09.2022**
(21) Application number: 20209335.7
(22) Date of filing: 23.11.2020
(51) Int. Cl.: A61F 2/66, A61F 2/50

(54) **A BIOINSPIRED MULTI-AXIAL ANKLE PROSTHESIS ENABLING ADJUSTABLE JOINT ORIENTATIONS BASED ON FIXED AXIS LENGTH**
BIOINSPIRIERTE MEHRACHSIGE KNÖCHELPROTHESE ZUR ERMÖGLICHUNG VON EINSTELLBAREN GELENKAUSRICHTUNGEN BASIEREND AUF EINER FESTEN ACHSENLÄNGE
PROTHÈSE DE CHEVILLE MULTI-AXIALE BIO-INSPIRÉE PERMETTANT D'AJUSTER L'ORIENTATION DE L'ARTICULATION EN FONCTION D'UNE LONGUEUR D'AXE FIXE

(30) Priority: 14.08.2020 CN 202010821143
(43) Date of publication of application: 16.02.2022
(73) Proprietor: Jilin University, Changchun, Jilin Province 130012 (CN)
(72) Inventor: REN, Lei, Changchun City Jilin Province 130022 (CN); WANG, Kunyang, Changchun City Jilin Province 130022 (CN); ZENG, Yi, Changchun City Jilin Province 130022 (CN); QIAN, Zhihui, Changchun City Jilin Province 130022 (CN); XIU, Haohua, Changchun City Jilin Province 130022 (CN); LIANG, Wei, Changchun City Jilin Province 130022 (CN); REN, Luquan, Changchun City Jilin Province 130022 (CN)
(74) Representative: Petculescu, Ana-Maria

(56) References cited:
- CN-A- 111 714 259
- US-A- 4 718 913
- US-A1- 2003 163 206

## Description

### FIELD OF THE INVENTION

The present disclosure belongs to the technical field of bionic human prostheses, and specifically relates to a bioinspired multi-axial ankle prosthesis enabling adjustable joint orientations based on fixed axis length. The invention is defined in the claims.

### BACKGROUND OF THE INVENTION

At present, ankle joint prosthesis products on the market are mainly designed with a single axis, while the human ankle joint axis is composed of multiple axes. Therefore, kinematic characteristics of the single-axis ankle joint prosthesis are quite different from those of healthy human limbs, which will have an influence on the experience of the wearer during use; specifically, the wearer feels somatosensory discomfort, has unnatural walking gait, and has low adaptability to inclined roads. There are also some multi-axis ankle joint prosthesis products on the market, but a spatial angle of two axes in these prostheses are fixed. For different amputees, the ankle joint angles of their healthy limbs are different, which leads to a personalized adaptation of such products and poor movement coordination of a limb on the affected side and a limb on the normal side of the wearer. US4718913 discloses a multi axial ankle prosthesis wherein an upper member represented by an upper ankle plate and lower member represented by a plantar plate are connected by springs.

### SUMMARY OF THE INVENTION

An object of the present disclosure is to provide a multi-axis ankle joint prosthesis product with an adjustable joint axis angle and based on a fixed axis length, which is of a multi-axis design and which can perform personalized adjustment on spatial angle and orientation of ankle joint movement axes according to biomechanical characteristics of ankle joints of different patients, thereby improving movement coordination of a limb on the affected side and a limb on the normal side of the wearer, improving the environmental adaptability and wearing comfort of the prosthesis, and coping with different road surface changes; moreover, the axis length is fixed, so that the structure is more stable, thus effectively reducing the wearer's energy consumption, and enabling the wearer to adjust the joint axis of the prosthesis through simple operations by himself/herself.

The present disclosure is composed of a left component A, a front component B, an upper cover C, a joint axis component D, a rear component E, a right component F, a bottom cover G, an auxiliary plate I 1, a spring I 2, a spring II 3, an auxiliary plate II 4, a spring III 5, and a spring IV 6; wherein a lower end of a left connecting plate 7 of the left component A is fixedly connected to an upper face on a left end of the bottom cover G, and a lower end of a right connecting plate 28 of the right component F is fixedly connected to an upper face on a right end of the bottom cover G; an approximately upper left side of a rear face of a front connecting plate 10 of the front component B is fixedly connected to an approximately upper left side of a front face of a rear connecting plate 27 of the rear component E by the auxiliary plate I 1, and an approximately upper right side of the rear face of the front connecting plate 10 of the front component B is fixedly connected to an approximately upper right side of the front face of the rear connecting plate 27 of the rear component E by the auxiliary plate II 4; an upper end of the front connecting plate 10 of the front component B is fixedly connected to a lower face on a front end of the upper cover C, and an upper end of the rear connecting plate 27 of the rear component E is fixedly connected to a lower face on a rear end of the upper cover C; the joint axis component D is located in a space enclosed by the left component A, the right component F, the upper cover C, the front component B, the rear component E, and the bottom cover G; a ring of a front link 17 of the joint axis component D is inserted in a slot IV q of a R30 slotted block I 11 of the front component B, and is fixedly connected to a R30 slot I q2 of the R30 slotted block I 11 by a bolt; a ring of a rear link 15 of the joint axis component D is inserted in a slot VI d1 of a R30 slotted block II 26 of the rear component E, and is fixedly connected to a R30 slot II d2 of the R30 slotted block II 26 by a bolt; a ring of a left link 18 of the joint axis component D is inserted in a slot II g of a R25 slotted block I 9 of the left component A, and is fixedly connected to a R25 slot I g2 of the R25 slotted block I 9 by a bolt; a ring of a right link 16 of the joint axis component D is inserted in a slot VII m1 of a R25 slotted block II 30 of the right component F, and is fixedly connected to a R25 slot II m2 of the R25 slotted block II 30 by a bolt; a left end of the spring I 2 is fixedly connected to a right side of a spring seat II h of the front connecting plate 10 of the front component B, and a right end of the spring I 2 is fixedly connected to a spring seat V g1 of the right connecting plate 28 of the right component F; a left end of the spring II 3 is fixedly connected to a spring seat I a of the left connecting plate 7 of the left component A, and a right end of the spring II 3 is fixedly connected to a left side of the spring seat II h of the front connecting plate 10 of the front component B; an upper end of the spring III 5 is fixedly connected to a spring seat IV z of the rear connecting plate 27 of the rear component E, and a lower end of the spring III 5 is fixedly connected to a spring seat VII s1 of the bottom cover G; an upper end of the spring IV 6 is fixedly connected to a spring seat III i of the front connecting plate 10 of the front component B, and a lower end of the spring IV 6 is fixedly connected to a spring seat VI r1 of the bottom cover G. After installation is completed, rings of the front link, rear link, left link, and right link of the joint axis component D are connected to the front component B, rear component E, left component A, and right component F respectively, and extreme ends of rods of the front link, rear link, left link, and right link contact with bottoms of front, rear, left, and right tubes respectively, so the front link, rear link, left link, and right link cannot slide along the tubes, and the overall axis length of the joint axis component D is fixed.

The left component A is composed of the left connecting plate 7, a vertical slotted block pair I 8 and the R25 slotted block I 9. A lower part of a right face of the left connecting plate 7 is provided with the spring seat I a at the center, and an upper part of the right face of the left connecting plate 7 is symmetrically provided with a straight slot II c and a straight slot I b on two sides in front of and behind the center; the vertical slotted block pair I 8 is composed of two front and rear slotted blocks having the same structure. Each of the slotted blocks is provided with a slot in the front-and-rear direction, and a left end of the slotted block is rectangular; the R25 slotted block I9 is provided with a column II f and a column I e on front and rear ends respectively; the R25 slotted block I 9 is provided with a R25 slot I g2 in the up-and-down direction; the R25 slotted block I 9 is provided with a slot II g in the left-and-right direction; the R25 slotted block I9 is located between the two front and rear slotted blocks of the vertical slotted block pair I 8; the column II f and the column I e of the R25 slotted block I 9 are slidingly connected to the slots of the two front and rear slotted blocks; left ends of the two slotted blocks of the vertical slotted block pair I 8 are respectively fixedly connected to the straight slot II c and the straight slot I b of the left connecting plate 7.

The front component B is composed of the front connecting plate 10, the R30 slotted block I 11, a vertical slotted block pair II 12, and a limiting plate I13. A lower end of a rear face of the front connecting plate 10 is provided at the center with a crossbar with the spring seat II h and the spring seat III i. An upper part of the rear face of the front connecting plate 10 is provided at the center with a hole I k, and left and right sides of the hole Ik are symmetrically provided with a straight slot IV1 and a straight slot IIIj; a center of an upper part of a front face of the front connecting plate 10 is fixedly connected with an outer seat I o at the position of the hole I k; left and right ends of the R30 slotted block I11 are respectively provided with a column IV s and a column III r; the R30 slotted block I 11 is provided with the R30 slot I q2 in the up-and-down direction; the R30 slotted block I 11 is provided with the slot IV q in the front-and-rear direction; the vertical slotted block pair II12 is composed of two left and right slotted blocks having the same structure. Each of the slotted blocks is provided with a slot in the left-and-right direction, and a rear end of the slotted block is rectangular; a front end of the limiting plate I 13 is provided with a threaded rod I u; left and right end plates of the limiting plate I 13 are symmetrically provided with an opened slot I t2 and an opened slot II t; a nut is placed on the outer seat I o; the threaded rod I u of the limiting plate I 13 is inserted into the hole I k of the front connecting plate 10 and the outer seat I o, and is fixedly connected to the nut on the outer seat I o; the R30 slotted block I 11 is located between the left and right end plates of the limiting plate I 13 and the two left and right slotted blocks of the vertical slotted block pair II 12, and the column IV s of the R30 slotted block I 11 is slidingly connected with the slot of the left slotted block of the vertical slotted block pair II 12 and the opened slot I t2 of the limiting plate I 13; the column III r of the R30 slotted block I 11 is slidingly connected with the slot of the right slotted block of the vertical slotted block pair II12 and the opened slot II t of the limiting plate I 13; and front ends of the two left and right slotted blocks of the vertical slotted block pair II12 are respectively fixedly connected with the straight slot IV 1 and the straight slot III j of the front connecting plate 10.

The joint axis component D is composed of an ankle joint axis assembly H, a subtalar joint axis assembly I, a bolt 14, the rear link 15, the right link 16, the front link 17 and the left link 18, wherein the ankle joint axis assembly H is composed of a right tube 19, a base I 20 and a left tube 21; the right tube 19, the base I 20 and the left tube 21 are fixedly connected in sequence; the base I 20 is provided with a through hole v at the center; the subtalar joint axis assembly I is composed of a rear tube 22 , a base II 23 and a front tube 24; the rear tube 22, the base II 23 and the front tube 24 are fixedly connected in sequence; the base II 23 is provided with a threaded hole w at the center; the right tube 19 and the left tube 21 are short tubes, and the rear tube 22 and the front tube 24 are long tubes; the base I 20 of the ankle joint axis assembly H is fixedly connected to the base II 23 of the subtalar joint axis assembly I by the bolt 14, and the left tube 21 and the right tube 19 of the ankle joint axis assembly H form a cross shape with the rear tube 22 and the front tube 24 of the subtalar joint axis assembly I; the rear link 15, the front link 17, the left link 18 and the right link 16 have the same structure, and they are each formed by connecting a rod x and a ring x1; the rod of the rear link 15 is in clearance fit with a rear portion of the rear tube 22 of the subtalar joint axis assembly I; the rod of the right link 16 is clearance fit with a right portion of the right tube 19 of the ankle joint axis assembly H; the rod of the front link 17 is clearance fit with a front portion of the front tube 26 of the subtalar joint axis assembly I; and the rod of the left link 18 is clearance fit with a left portion of the left tube 21 of the ankle joint axis assembly H.

The rear component E is composed of a vertical slotted block pair III 25, the R30 slotted block II 26, and the rear connecting plate 27, wherein the vertical slotted block pair III 25 is composed of two left and right slotted blocks having the same structure. Each of the slotted blocks is provided with a slot in the left-and-right direction, and a rear end of the slotted block is rectangular; the R30 slotted block II 26 is provided with a column VI f1 and a column V e1 on left and right ends respectively; the R30 slotted block II 30 is provided with the R30 slot II d2 in the up-and-down direction; the R30 slotted block II 30 is provided with the slot VI d1 in the front-and-rear direction; a lower end of a front face of the rear connecting plate 27 is provided at the center with a crossbar with the spring seat IV z; an approximately lower end of the front face of the rear connecting plate 27 is symmetrically provided with a straight slot VI b1 and a straight slot V a1 in the left-and-right direction; the R30 slotted block II 26 is located between the two left and right slotted blocks of the vertical slotted block pair III 25; the column VI f1 and the column V e1 of the R30 slotted block II 26 are slidingly connected to the slots of the two left and right slotted blocks; and rear ends of the two left and right slotted blocks of the vertical slotted block pair III 25 are respectively fixedly connected to the straight slot VI b1 and the straight slot V a1 of the rear connecting plate 27.

The right component F is composed of the right connecting plate 28, a vertical slotted block pair IV 29, the R25 slotted block II 30, and a limiting plate II 3 1. A lower portion of the right connecting plate 28 is provided with the spring seat V g1 at the center, and an upper portion of the right connecting plate 28 is provided with a hole II h1 at the center, which is symmetrically provided with a straight slot VII i1 and a straight slot VIII j 1 on two front and rear sides; a center of an upper part of a right face of the right connecting plate 28 is fixedly connected with an outer seat II k1 at the position of the hole II h1; the vertical slotted block pair IV 29 is composed of two front and rear slotted blocks having the same structure. Each of the slotted blocks is provided with a slot in the front-and-rear direction, and a right end of the slotted block is rectangular. Front and rear ends of the R25 slotted block II 30 are respectively provided with a column VII11 and a column VIII n1; the R25 slotted block II 30 is provided with the R25 slot II m2 in the up-and-down direction; the R25 slotted block II 30 is provided with the slot VII m1 in the left-and-right direction. A right face of the limiting plate II 31 is provided with a threaded rod II q1 at the center; front and rear plates of the limiting plate II 31 are symmetrically provided with an opened slot III p1 and an opened slot IV p2; a nut is placed on the outer seat II k1; the threaded rod II q1 of the limiting plate II 31 is inserted into the hole II h1of the right connecting plate 28 and the outer seat II k1, and is fixedly connected to the nut on the outer seat II k1. Right ends of the two front and rear slotted blocks of the vertical slotted block pair IV 29 are fixedly connected to the straight slot VII i1 and the straight slot VIII j1 of the right connecting plate 28 respectively; the R25 slotted block II 30 is located between the front and rear plates of the limiting plate II 31 and the two front and rear slotted blocks of the vertical slotted block pair IV 29, and the column III l1 of the R25 slotted block II 30 is slidingly connected with the slot of the front slotted block of the vertical slotted block pair IV 29 and the opened slot III p1 of the limiting plate II 31; and the column VIII n1 of the R25 slotted block II 30 is slidingly connected with the slot of the rear slotted block of the vertical slotted block pair IV 29 and the opened slot IV p2 of the limiting plate II 31.

The bottom cover G is provided with the spring seat VIr1 located forwardly on a transverse central line in the front-and-rear direction on an upper face thereof, and the spring seat VII s1 located rearwardly on the transverse central line in the front-and-rear direction on the upper face thereof.

The process of adjusting the joint axis angle of the present disclosure is described as follows:
1. The upper cover C is opened and the bolt 14 is loosened.
2. The bolt on the R30 slotted block I 11 in the front component B remains in a tightened state, and the bolt on the R30 slotted block II 26 in the rear component E remains in a tightened state, so that the front link 17 and the rear link 15 inserted in the slot IV q and the slot VI d1 are fixed, thereby fixing the position of the subtalar joint axis assembly I.
3. The bolt on the R25 slotted block I9 in the left component A is loosened, the bolt on the R25 slotted block II 30 in the right component F is loosened, the ankle joint axis assembly H is rotated, and a projection angle of the ankle joint axis assembly H on the horizontal plane is adjusted. After the adjustment is completed, the bolts on the R25 slotted block I 9 and the R25 slotted block II 30 are re-tightened.
4. The nut on the outer seat II k1 of the right connecting plate 28 in the right component F is rotated to drive the limiting plate II 31 to slide along the hole II h1, thereby driving the R25 slotted block II 30 to slide along the slot VIII o1 of the vertical slotted block pair IV 29, thereby driving the ankle joint axis assembly H to rotate to adjust the projection angle of the ankle joint axis assembly H on the vertical plane. After the adjustment is completed, the nut on the outer seat II k1 will not be rotated. Hitherto, the angle adjustment of the ankle joint axis assembly H is completed.
5. The bolt on the R30 slotted block I 11 in the front component B is loosened, the bolt on the R30 slotted block II 26 in the rear component E is loosened, the subtalar joint axis assembly I is rotated, and a projection angle of the subtalar joint axis assembly I on the horizontal plane is adjusted. After the adjustment is completed, the bolts on the R30 slotted block I 11 and the R30 slotted block II 26 are re-tightened.
6. The nut on the outer seat I o of the front connecting plate 10 in the front component B is rotated to drive the limiting plate I 13 to slide along the hole I k, thereby driving the R30 slotted block I 11 to slide along the slot III p of the vertical slotted block pair II 12, thereby driving the subtalar joint axis assembly I to rotate to adjust the projection angle of the subtalar joint axis assembly I on the vertical plane. After the adjustment is completed, the nut on the outer seat I o will not be rotated. Hitherto, the angle adjustment of the subtalar joint axis assembly I is completed.
7. After the adjustments of the ankle joint axis assembly H and the subtalar joint axis assembly I are completed, the bolt 14 is tightened so that the ankle joint axis assembly H and the subtalar joint axis assembly I cannot rotate relative to each other, and the upper cover C is closed.

Hitherto, the adjustment of the joint axis angle is completed.

The present disclosure has the following advantageous effects.

The present disclosure can first measure the joint angle at the ankle joint on the healthy limb side of the human body, then adjust the angle of the joint axis of the prosthesis product by itself, and then fine-tune the angle of the prosthesis joint axis according to the user's own wearing experience until the optimal wearing and using effect is achieved. Due to the fixed axis length, the product is more stable during use, less prone to swaying, and simple in structure. Part of the adjustment only needs the rotation of the nut disposed on the outside, so the adjustment is made more simple and convenient. The present disclosure can greatly alleviate the problems of low fit degree, poor adaptability and high energy consumption for the wearer in ankle joint prosthesis products.

### BRIEF DESCRIPTION OF THE DRAWINGS

FIG. 1 is a front perspective view of a bioinspired multi-axial ankle prosthesis enabling adjustable joint orientations based on fixed axis length.
FIG. 2 is a rear perspective view of a bioinspired multi-axial ankle prosthesis enabling adjustable joint orientations based on fixed axis length.
FIG. 3 is a perspective view of a left component A.
FIG. 4 is a perspective view of a left connecting plate 7.
FIG. 5 is a perspective view of a vertical slotted block of a vertical slotted block pair I 8.
FIG. 6 is a perspective view of a R25 slotted block I.
FIG. 7 is a perspective view of a front component B.
FIG. 8 is a right side view of the front component B.
FIG. 9 is a rear perspective view of a front connecting plate 10.
FIG. 10 is a front perspective view of the front connecting plate 10.
FIG. 11 is a perspective view of a vertical slotted block of a vertical slotted block pair II 12.
FIG. 12 is a perspective view of a R30 slotted block I 11.
FIG. 13 is a perspective view of a limiting plate I 13.
FIG. 14 is a perspective view of a joint axis component D.
FIG. 15 is a perspective view of an ankle joint axis assembly H.
FIG. 16 is a perspective view of a subtalar joint axis assembly I.
FIG. 17 is a perspective view of a link.
FIG. 18 is a perspective view of a rear component E.
FIG. 19 is a perspective view of a rear connecting plate 27.
FIG. 20 is a perspective view of a vertical slotted block of a vertical slotted block pair III 25.
FIG. 21 is a perspective view of a R30 slotted block II 26.
FIG. 22 is a perspective view of a right component F.
FIG. 23 is a front view of the right component F.
FIG. 24 is a left perspective view of a right connecting plate 28.
FIG. 25 is a right perspective view of the right connecting plate 28.
FIG. 26 is a perspective view of a R25 slotted block II 30.
FIG. 27 is a perspective view of a vertical slotted block of a vertical slotted block pair IV 29.
FIG. 28 is a perspective view of a limiting plate II 30.
FIG. 29 is a perspective view of a bottom cover G.
FIG. 30 is a perspective view of an auxiliary plate I 1.
FIG. 31 is a perspective view of an auxiliary plate II 4.
wherein: A: left component; B: front component; C: upper cover; D: joint axis component; E: rear component; F: right component; G: bottom cover; H: ankle joint axis assembly; I: subtalar joint axis assembly; 1: auxiliary plate I; 2: spring I; 3: spring II; 4: auxiliary plate II; 5: spring III; 6: spring IV; 7: left connecting plate; 8: vertical slotted block pair I; 9: R25 slotted block I; 10: front connecting plate; 11: R30 slotted block I; 12: vertical slotted block pair II; 13: limiting plate I; 14: bolt; 15: rear link; 16: right link; 17: front link; 18: left link; 19: right tube; 20: base I; 21: left tube; 22: rear tube; 23: base II; 24: front tube; 25: vertical slotted block pair III; 26: R30 slotted block II; 27: rear connecting plate; 28: right connecting plate; 29: vertical slotted block pair IV; 30: R25 slotted block II; 31: limiting plate II; a: spring seat I; b: straight slot I; c: straight slot II; d: slot I; e: column I; f: column II; g: slot II; g2: R25 slot I; h: spring seat II; i: spring seat III; j: straight slot III; k: hole I; 1: straight slot IV; o: outer seat I; p: slot III; q: slot IV; q2: R30 slot I; r: column III; s: column IV; t: opened slot II; t2: opened slot I; u: threaded rod I; v: through hole; w: threaded hole; x: rod; x1: ring; z: spring seat IV; a1: straight slot V; b1: straight slot VI; c1: slot V; d1: slot VI; d2: R30 slot II; e1: column V; f1: column VI; g1: spring seat V; h1: hole II; i1: straight slot VII; j1: straight slot VIII; k1: outer seat II; l1: column VII; m1: slot VII; m2: R25 slot II; n1: column VIII; o1: slot VIII; p1: opened slot III; p2: opened slot IV; q1: threaded rod II; r1: spring seat VI; s1: spring seat VII.

### DETAILED DESCRIPTION OF THE EMBODIMENT(S) OF THE INVENTION

The present disclosure will be described below in conjunction with the drawings.

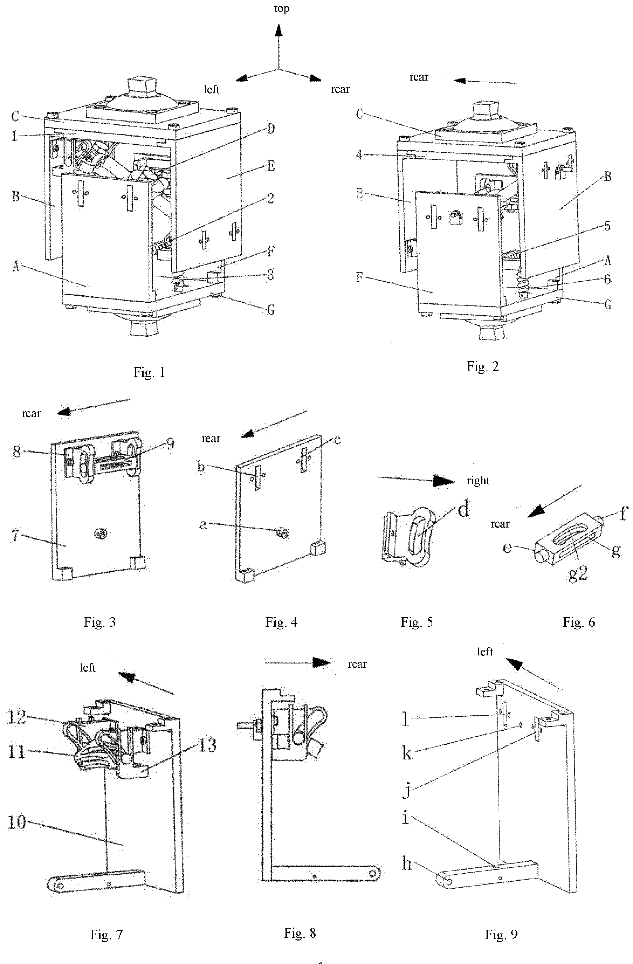

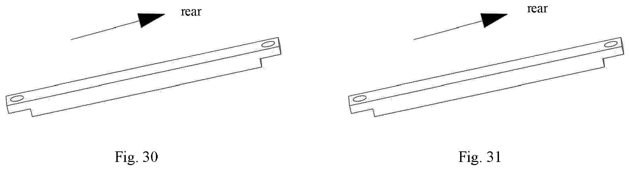

As shown in FIGS. 1, 2, 30 and 31, the present disclosure is composed of a left component A, a front component B, an upper cover C, a joint axis component D, a rear component E, a right component F, a bottom cover G, an auxiliary plate I 1, a spring I 2, a spring II 3, an auxiliary plate II 4, a spring III 5, and a spring IV 6; wherein a lower end of a left connecting plate 7 of the left component A is fixedly connected to an upper face on a left end of the bottom cover G, and a lower end of a right connecting plate 28 of the right component F is fixedly connected to an upper face on a right end of the bottom cover G; an approximately upper left side of a rear face of a front connecting plate 10 of the front component B is fixedly connected to an approximately upper left side of a front face of a rear connecting plate 27 of the rear component E by the auxiliary plate I 1, and an approximately upper right side of the rear face of the front connecting plate 10 of the front component B is fixedly connected to an approximately upper right side of the front face of the rear connecting plate 27 of the rear component E by the auxiliary plate II 4; an upper end of the front connecting plate 10 of the front component B is fixedly connected to a lower face on a front end of the upper cover C, and an upper end of the rear connecting plate 27 of the rear component E is fixedly connected to a lower face on a rear end of the upper cover C; the joint axis component D is located in a space enclosed by the left component A, the right component F, the upper cover C, the front component B, the rear component E, and the bottom cover G; a ring of a front link 17 of the joint axis component D is inserted in a slot IV q of a R30 slotted block I 11 of the front component B, and is fixedly connected to a R30 slot I q2 of the R30 slotted block I 11 by a bolt; a ring of a rear link 15 of the joint axis component D is inserted in a slot VI d1 of a R30 slotted block II 26 of the rear component E, and is fixedly connected to a R30 slot II d2 of the R30 slotted block II 26 by a bolt; a ring of a left link 18 of the joint axis component D is inserted in a slot II g of a R25 slotted block I 9 of the left component A, and is fixedly connected to a R25 slot I g2 of the R25 slotted block I 9 by a bolt; a ring of a right link 16 of the joint axis component D is inserted in a slot VII m1 of a R25 slotted block II30 of the right component F, and is fixedly connected to a R25 slot II m2 of the R25 slotted block II 30 by a bolt; a left end of the spring I 2 is fixedly connected to a right side of a spring seat II h of the front connecting plate 10 of the front component B, and a right end of the spring I 2 is fixedly connected to a spring seat V g1 of the right connecting plate 28 of the right component F; a left end of the spring II 3 is fixedly connected to a spring seat I a of the left connecting plate 7 of the left component A, and a right end of the spring II 3 is fixedly connected to a left side of the spring seat II h of the front connecting plate 10 of the front component B; an upper end of the spring III 5 is fixedly connected to a spring seat IV z of the rear connecting plate 27 of the rear component E, and a lower end of the spring III5 is fixedly connected to a spring seat VII s1 of the bottom cover G; an upper end of the spring IV 6 is fixedly connected to a spring seat III i of the front connecting plate 10 of the front component B, and a lower end of the spring IV 6 is fixedly connected to a spring seat VI r1 of the bottom cover G. After installation is completed, rings of the front link, rear link, left link, and right link of the joint axis component D are connected to the front component B, rear component E, left component A, and right component F respectively, and extreme ends of rods of the front link, rear link, left link, and right link contact with bottoms of front, rear, left, and right tubes respectively, so the front link, rear link, left link, and right link cannot slide along the tubes, and the overall axis length of the joint axis component D is fixed.

As shown in FIGS. 3 to 6, the left component A is composed of the left connecting plate 7, a vertical slotted block pair I 8 and the R25 slotted block I 9. A lower part of a right face of the left connecting plate 7 is provided with the spring seat I a at the center, and an upper part of the right face of the left connecting plate 7 is symmetrically provided with a straight slot II c and a straight slot I b on two sides in front of and behind the center; the vertical slotted block pair I 8 is composed of two front and rear slotted blocks having the same structure. Each of the slotted blocks is provided with a slot in the front-and-rear direction, and a left end of the slotted block is rectangular; the R25 slotted block 19 is provided with a column II f and a column I e on front and rear ends respectively; the R25 slotted block I 9 is provided with a R25 slot I g2 in the up-and-down direction; the R25 slotted block I 9 is provided with a slot II g in the left-and-right direction; the R25 slotted block I 9 is located between the two front and rear slotted blocks of the vertical slotted block pair I 8; the column II f and the column I e of the R25 slotted block I 9 are slidingly connected to the slots of the two front and rear slotted blocks; left ends of the two slotted blocks of the vertical slotted block pair I 8 are respectively fixedly connected to the straight slot II c and the straight slot I b of the left connecting plate 7.

As shown in FIGS. 7 to 13, the front component B is composed of the front connecting plate 10, the R30 slotted block I 11, a vertical slotted block pair II 12, and a limiting plate I 13. A lower end of a rear face of the front connecting plate 10 is provided at the center with a crossbar with the spring seat II h and the spring seat III i. An upper part of the rear face of the front connecting plate 10 is provided at the center with a hole I k, and left and right sides of the hole I k are symmetrically provided with a straight slot IV 1 and a straight slot III j; a center of an upper part of a front face of the front connecting plate 10 is fixedly connected with an outer seat I o at the position of the hole I k; left and right ends of the R30 slotted block I11 are respectively provided with a column IV s and a column III r; the R30 slotted block I 11 is provided with the R30 slot I q2 in the up-and-down direction; the R30 slotted block I 11 is provided with the slot IV q in the front-and-rear direction; the vertical slotted block pair II12 is composed of two left and right slotted blocks having the same structure. Each of the slotted blocks is provided with a slot in the left-and-right direction, and a rear end of the slotted block is rectangular; a front end of the limiting plate I 13 is provided with a threaded rod I u; left and right end plates of the limiting plate I 13 are symmetrically provided with an opened slot I t2 and an opened slot II t; a nut is placed on the outer seat I o; the threaded rod I u of the limiting plate I 13 is inserted into the hole I k of the front connecting plate 10 and the outer seat I o, and is fixedly connected to the nut on the outer seat I o; the R30 slotted block I 11 is located between the left and right end plates of the limiting plate I 13 and the two left and right slotted blocks of the vertical slotted block pair II 12, and the column IV s of the R30 slotted block I11 is slidingly connected with the slot of the left slotted block of the vertical slotted block pair II 12 and the opened slot It2 of the limiting plate I 13; the column III r of the R30 slotted block I 11 is slidingly connected with the slot of the right slotted block of the vertical slotted block pair II12 and the opened slot II t of the limiting plate I 13; and front ends of the two left and right slotted blocks of the vertical slotted block pair II 12 are respectively fixedly connected with the straight slot IV 1 and the straight slot III j of the front connecting plate 10.

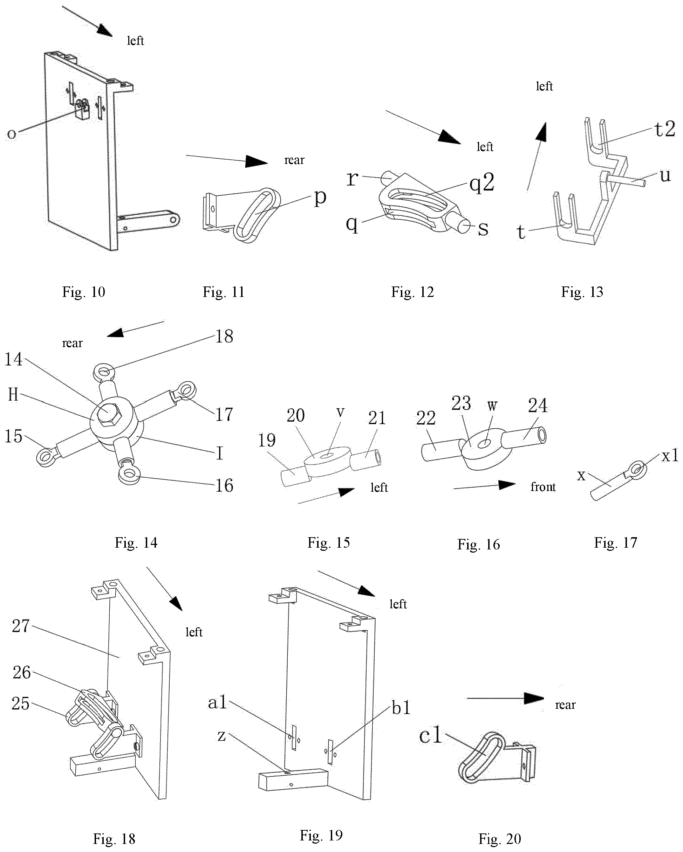

As shown in FIGS. 14 to 17, the joint axis component D is composed of an ankle joint axis assembly H, a subtalar joint axis assembly I, a bolt 14, the rear link 15, the right link 16, the front link 17 and the left link 18, wherein the ankle joint axis assembly H is composed of a right tube 19, a base I 20 and a left tube 21; the right tube 19, the base I 20 and the left tube 21 are fixedly connected in sequence; the base I 20 is provided with a through hole v at the center; the subtalar joint axis assembly I is composed of a rear tube 22 , a base II 23 and a front tube 24; the rear tube 22, the base II 23 and the front tube 24 are fixedly connected in sequence; the base II23 is provided with a threaded hole w at the center; the right tube 19 and the left tube 21 are short tubes, and the rear tube 22 and the front tube 24 are long tubes; the base I 20 of the ankle joint axis assembly H is fixedly connected to the base II 23 of the subtalar joint axis assembly I by the bolt 14, and the left tube 21 and the right tube 19 of the ankle joint axis assembly H form a cross shape with the rear tube 22 and the front tube 24 of the subtalar joint axis assembly I; the rear link 15, the front link 17, the left link 18 and the right link 16 have the same structure, and they are each formed by connecting a rod x and a ring x1; the rod of the rear link 15 is in clearance fit with a rear portion of the rear tube 22 of the subtalar joint axis assembly I; the rod of the right link 16 is clearance fit with a right portion of the right tube 19 of the ankle joint axis assembly H; the rod of the front link 17 is clearance fit with a front portion of the front tube 26 of the subtalar joint axis assembly I; and the rod of the left link 18 is clearance fit with a left portion of the left tube 21 of the ankle joint axis assembly H.

As shown in FIGS. 18 to 21, the rear component E is composed of a vertical slotted block pair III 25, the R30 slotted block II 26, and the rear connecting plate 27, wherein the vertical slotted block pair III 25 is composed of two left and right slotted blocks having the same structure. Each of the slotted blocks is provided with a slot in the left-and-right direction, and a rear end of the slotted block is rectangular; the R30 slotted block II 26 is provided with a column VI f1 and a column V e1 on left and right ends respectively; the R30 slotted block II 30 is provided with the R30 slot II d2 in the up-and-down direction; the R30 slotted block II 30 is provided with the slot VI d1 in the front-and-rear direction; a lower end of a front face of the rear connecting plate 27 is provided at the center with a crossbar with the spring seat IV z; an approximately lower end of the front face of the rear connecting plate 27 is symmetrically provided with a straight slot VI b1 and a straight slot V a1 in the left-and-right direction; the R30 slotted block II 26 is located between the two left and right slotted blocks of the vertical slotted block pair III 25; the column VI f1 and the column V e1 of the R30 slotted block II 26 are slidingly connected to the slots of the two left and right slotted blocks; and rear ends of the two left and right slotted blocks of the vertical slotted block pair III 25 are respectively fixedly connected to the straight slot VI b1 and the straight slot V a1 of the rear connecting plate 27.

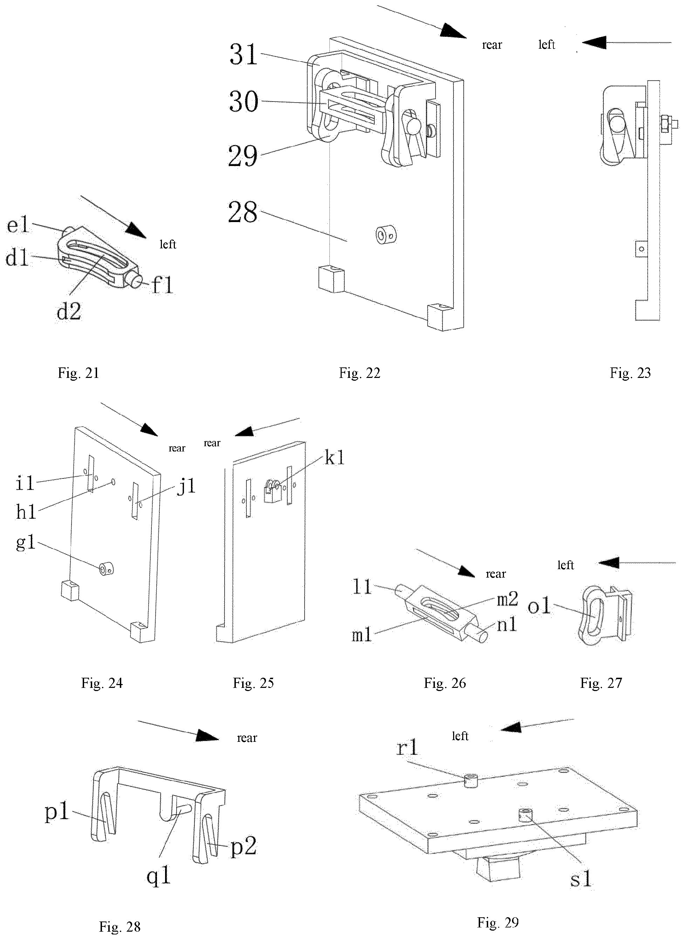

As shown in FIGS. 22 to 28, the right component F is composed of the right connecting plate 28, a vertical slotted block pair IV 29, the R25 slotted block II 30, and a limiting plate II 3 1. A lower portion of the right connecting plate 28 is provided with the spring seat V g1 at the center, and an upper portion of the right connecting plate 28 is provided with a hole II h1 at the center, which is symmetrically provided with a straight slot VII i 1 and a straight slot VIII j 1 on two front and rear sides; a center of an upper part of a right face of the right connecting plate 28 is fixedly connected with an outer seat II k1 at the position of the hole II h1; the vertical slotted block pair IV 29 is composed of two front and rear slotted blocks having the same structure. Each of the slotted blocks is provided with a slot in the front-and-rear direction, and a right end of the slotted block is rectangular. Front and rear ends of the R25 slotted block II 30 are respectively provided with a column VII11 and a column VIII n1; the R25 slotted block II 30 is provided with the R25 slot II m2 in the up-and-down direction; the R25 slotted block II 30 is provided with the slot VII m1 in the left-and-right direction. A right face of the limiting plate II 31 is provided with a threaded rod II q1 at the center; front and rear plates of the limiting plate II 31 are symmetrically provided with an opened slot III p1 and an opened slot IV p2; a nut is placed on the outer seat II k1; the threaded rod II q1 of the limiting plate II 31 is inserted into the hole II h1 of the right connecting plate 28 and the outer seat II k1, and is fixedly connected to the nut on the outer seat II k1. Right ends of the two front and rear slotted blocks of the vertical slotted block pair IV 29 are fixedly connected to the straight slot VII i1 and the straight slot VIII j1 of the right connecting plate 28 respectively; the R25 slotted block II 30 is located between the front and rear plates of the limiting plate II 31 and the two front and rear slotted blocks of the vertical slotted block pair IV 29, and the column III 11 of the R25 slotted block II 30 is slidingly connected with the slot of the front slotted block of the vertical slotted block pair IV 29 and the opened slot III p1 of the limiting plate II 31; and the column VIII n1 of the R25 slotted block II 30 is slidingly connected with the slot of the rear slotted block of the vertical slotted block pair IV 29 and the opened slot IV p2 of the limiting plate II 3 1.

As shown in FIG. 29, the bottom cover G is provided with the spring seat VI r1 located forwardly on a transverse central line in the front-and-rear direction on an upper face thereof, and the spring seat VII s1 located rearwardly on the transverse central line in the front-and-rear direction on the upper face thereof.

## Claims

1. A bioinspired multi-axial ankle prosthesis enabling adjustable joint orientations based on fixed axis length, wherein the bioinspired multi-axial ankle prosthesis enabling adjustable joint orientations based on fixed axis length is composed of a left component (A), a front component (B), an upper cover (C), a joint axis component (D), a rear component (E), a right component (F), a bottom cover (G), an auxiliary plate I (1), a spring I (2), a spring II (3), an auxiliary plate II (4), a spring III (5), and a spring IV (6);
wherein a lower end of a left connecting plate (7) of the left component (A) is fixedly connected to an upper face on a left end of the bottom cover (G), and a lower end of a right connecting plate (28) of the right component (F) is fixedly connected to an upper face on a right end of the bottom cover (G);
an approximately upper left side of a rear face of a front connecting plate (10) of the front component (B) is fixedly connected to an approximately upper left side of a front face of a rear connecting plate (27) of the rear component (E) by the auxiliary plate I (1), and an approximately upper right side of the rear face of the front connecting plate (10) of the front component (B) is fixedly connected to an approximately upper right side of the front face of the rear connecting plate (27) of the rear component (E) by the auxiliary plate II (4);
an upper end of the front connecting plate (10) of the front component (B) is fixedly connected to a lower face on a front end of the upper cover (C), and an upper end of the rear connecting plate (27) of the rear component (E) is fixedly connected to a lower face on a rear end of the upper cover (C);
the joint axis component (D) is located in a space enclosed by the left component (A), the right component (F), the upper cover (C), the front component (B), the rear component (E), and the bottom cover (G);
a ring of a front link (17) of the joint axis component (D) is inserted in a slot IV (q) of a R30 slotted block I (11) of the front component (B), and is fixedly connected to a R30 slot I (q2) of the R30 slotted block I (11) by a bolt; a ring of a rear link (15) of the joint axis component (D) is inserted in a slot VI (d1) of a R30 slotted block II (26) of the rear component (E), and is fixedly connected to a R30 slot II (d2) of the R30 slotted block II (26) by a bolt; a ring of a left link (18) of the joint axis component (D) is inserted in a slot II (g) of a R25 slotted block I (9) of the left component (A), and is fixedly connected to a R25 slot I (g2) of the R25 slotted block I (9) by a bolt; and a ring of a right link (16) of the joint axis component (D) is inserted in a slot VII (m1) of a R25 slotted block II (30) of the right component (F), and is fixedly connected to a R25 slot II (m2) of the R25 slotted block II (30) by a bolt;
a left end of the spring I (2) is fixedly connected to a right side of a spring seat II (h) of the front connecting plate (10) of the front component (B), and a right end of the spring I (2) is fixedly connected to a spring seat V (g1) of the right connecting plate (28) of the right component (F);
a left end of the spring II (3) is fixedly connected to a spring seat I (a) of the left connecting plate (7) of the left component (A), and a right end of the spring II (3) is fixedly connected to a left side of the spring seat II (h) of the front connecting plate II (10) of the front component II (B);
an upper end of the spring III (5) is fixedly connected to a spring seat IV (z) of the rear connecting plate (27) of the rear component (E), and a lower end of the spring III (5) is fixedly connected to a spring seat VII (s1) of the bottom cover (G); and
an upper end of the spring IV (6) is fixedly connected to a spring seat III (i) of the front connecting plate (10) of the front component (B), and a lower end of the spring IV (6) is fixedly connected to a spring seat VI (r1) of the bottom cover (G).

2. The bioinspired multi-axial ankle prosthesis enabling adjustable joint orientations based on fixed axis length according to claim 1, wherein left component (A) is composed of the left connecting plate (7), a vertical slotted block pair I (8) and the R25 slotted block I (9);
a lower part of a right face of the left connecting plate (7) is provided with the spring seat I (a) at the center, and an upper part of the right face of the left connecting plate (7) is symmetrically provided with a straight slot II (c) and a straight slot I (b) on two sides in front of and behind the center;
the vertical slotted block pair I (8) is composed of two front and rear slotted blocks having the same structure; each of the slotted blocks is provided with a slot in the front-and-rear direction, and a left end of the slotted block is rectangular;
the R25 slotted block I (9) is provided with a column II (f) and a column I (e) on front and rear ends respectively; the R25 slotted block I (9) is provided with a R25 slot I (g2) in the up-and-down direction; the R25 slotted block I (9) is provided with a slot II (g) in the left-and-right direction; the R25 slotted block I (9) is located between the two front and rear slotted blocks of the vertical slotted block pair I (8); the column II (f) and the column I (e) of the R25 slotted block I (9) are slidingly connected to the slots of the two front and rear slotted blocks; and left ends of the two slotted blocks of the vertical slotted block pair I (8) are respectively fixedly connected to the straight slot II (c) and the straight slot I (b) of the left connecting plate (7).

3. The bioinspired multi-axial ankle prosthesis enabling adjustable joint orientations based on fixed axis length according to claim 1, wherein the front component (B) is composed of the front connecting plate (10), the R30 slotted block I (11), a vertical slotted block pair II (12), and a limiting plate I (13);
a lower end of a rear face of the front connecting plate (10) is provided at the center with a crossbar with the spring seat II (h) and the spring seat III (i), an upper part of the rear face of the front connecting plate (10) is provided at the center with a hole I (k), and left and right sides of the hole I (k) are symmetrically provided with a straight slot IV (1) and a straight slot III (j);
a center of an upper part of a front face of the front connecting plate (10) is fixedly connected with an outer seat I (o) at the position of the hole I (k); left and right ends of the R30 slotted block I (11) are respectively provided with a column IV (s) and a column III (r);
the R30 slotted block I (11) is provided with the R30 slot I (q2) in the up-and-down direction; the R30 slotted block I (11) is provided with the slot IV (q) in the front-and-rear direction;
the vertical slotted block pair II (12) is composed of two left and right slotted blocks having the same structure; each of the slotted blocks is provided with a slot in the left-and-right direction, and a rear end of the slotted block is rectangular;
a front end of the limiting plate I (13) is provided with a threaded rod I (u); left and right end plates of the limiting plate I (13) are symmetrically provided with an opened slot I (t2) and an opened slot II (t); a nut is placed on the outer seat I (o); the threaded rod I (u) of the limiting plate I (13) is inserted into the hole I (k) of the front connecting plate (10) and the outer seat I (o), and is fixedly connected to the nut on the outer seat I (o);
the R30 slotted block I (11) is located between the left and right end plates of the limiting plate I (13) and the two left and right slotted blocks of the vertical slotted block pair II (12), and the column IV (s) of the R30 slotted block I (11) is slidingly connected with the slot of the left slotted block of the vertical slotted block pair II (12) and the opened slot I (t2) of the limiting plate I (13); the column III (r) of the R30 slotted block I (11) is slidingly connected with the slot of the right slotted block of the vertical slotted block pair II (12) and the opened slot II (t) of the limiting plate I (13); and front ends of the two left and right slotted blocks of the vertical slotted block pair II (12) are respectively fixedly connected with the straight slot IV (1) and the straight slot III (j) of the front connecting plate (10).

4. The bioinspired multi-axial ankle prosthesis enabling adjustable joint orientations based on fixed axis length according to claim 1, wherein the joint axis component (D) is composed of an ankle joint axis assembly (H), a subtalar joint axis assembly (I), a bolt (14), the rear link (15), the right link (16), the front link (17) and the left link (18);
wherein the ankle joint axis assembly (H) is composed of a right tube (19), a base I (20) and a left tube (21); the right tube (19), the base I (20) and the left tube (21) are fixedly connected in sequence; the base I (20) is provided with a through hole (v) at the center; the subtalar joint axis assembly (I) is composed of a rear tube (22), a base II (23) and a front tube (24); the rear tube (22), the base II (23) and the front tube (24) are fixedly connected in sequence; the base II (23) is provided with a threaded hole (w) at the center; the right tube (19) and the left tube (21) are short tubes, and the rear tube (22) and the front tube (24) are long tubes; the base I (20) of the ankle joint axis assembly (H) is fixedly connected to the base II (23) of the subtalar joint axis assembly (I) by the bolt (14), and the left tube (21) and the right tube (19) of the ankle joint axis assembly (H) form a cross shape with the rear tube (22) and the front tube (24) of the subtalar joint axis assembly (I); the rear link (15), the front link (17), the left link (18) and the right link (16) have the same structure, and they are each formed by connecting a rod (x) and a ring (x1); the rod of the rear link (15) is in clearance fit with a rear portion of the rear tube (22) of the subtalar joint axis assembly (I); the rod of the right link (16) is clearance fit with a right portion of the right tube (19) of the ankle joint axis assembly (H); the rod of the front link (17) is clearance fit with a front portion of the front tube (26) of the subtalar joint axis assembly (I); and the rod of the left link (18) is clearance fit with a left portion of the left tube (21) of the ankle joint axis assembly (H).

5. The bioinspired multi-axial ankle prosthesis enabling adjustable joint orientations based on fixed axis length according to claim 1, wherein the rear component (E) is composed of a vertical slotted block pair III (25), the R30 slotted block II (26), and the rear connecting plate 27;
wherein the vertical slotted block pair III (25) is composed of two left and right slotted blocks having the same structure; each of the slotted blocks is provided with a slot in the left-and-right direction, and a rear end of the slotted block is rectangular;
the R30 slotted block II (26) is provided with a column VI (f1) and a column V (e1) on left and right ends respectively; the R30 slotted block II (30) is provided with the R30 slot II (d2) in the up-and-down direction; the R30 slotted block II (30) is provided with the slot VI (d1) in the front-and-rear direction; a lower end of a front face of the rear connecting plate (27) is provided at the center with a crossbar with the spring seat IV (z); an approximately lower end of the front face of the rear connecting plate (27) is symmetrically provided with a straight slot VI (b1) and a straight slot V (a1) in the left-and-right direction; the R30 slotted block II (26) is located between the two left and right slotted blocks of the vertical slotted block pair III (25); the column VI (f1) and the column V (e1) of the R30 slotted block II (26) are slidingly connected to the slots of the two left and right slotted blocks; and rear ends of the two left and right slotted blocks of the vertical slotted block pair III (25) are respectively fixedly connected to the straight slot VI (b1) and the straight slot V (a1) of the rear connecting plate (27).

6. The bioinspired multi-axial ankle prosthesis enabling adjustable joint orientations based on fixed axis length according to claim 1, wherein the right component (F) is composed of the right connecting plate (28), a vertical slotted block pair IV (29), the R25 slotted block II (30), and a limiting plate II (31);
a lower portion of the right connecting plate (28) is provided with the spring seat V (g1) at the center, and an upper portion of the right connecting plate (28) is provided with a hole II (h1) at the center, which is symmetrically provided with a straight slot VII (i1) and a straight slot VIII (j1) on two front and rear sides; a center of an upper part of a right face of the right connecting plate (28) is fixedly connected with an outer seat II (k1) at the position of the hole II (h1);
the vertical slotted block pair IV (29) is composed of two front and rear slotted blocks having the same structure; each of the slotted blocks is provided with a slot in the front-and-rear direction, and a right end of the slotted block is rectangular;
front and rear ends of the R25 slotted block II (30) are respectively provided with a column VII (11) and a column VIII (n1); the R25 slotted block II (30) is provided with the R25 slot II (m2) in the up-and-down direction; the R25 slotted block II (30) is provided with the slot VII (m1) in the left-and-right direction;
a right face of the limiting plate II (31) is provided with a threaded rod II (q1) at the center; front and rear plates of the limiting plate II (31) are symmetrically provided with an opened slot III (p1) and an opened slot IV (p2); a nut is placed on the outer seat II (k1); the threaded rod II (q1) of the limiting plate II (31) is inserted into the hole II (h1) of the right connecting plate (28) and the outer seat II (k1), and is fixedly connected to the nut on the outer seat II (k1);
right ends of the two front and rear slotted blocks of the vertical slotted block pair IV (29) are fixedly connected to the straight slot VII (i1) and the straight slot VIII (j1) of the right connecting plate (28) respectively; the R25 slotted block II (30) is located between the front and rear plates of the limiting plate II (31) and the two front and rear slotted blocks of the vertical slotted block pair IV (29), and the column III (11) of the R25 slotted block II (30) is slidingly connected with the slot of the front slotted block of the vertical slotted block pair IV (29) and the opened slot III (p1) of the limiting plate II (31); and the column VIII (n1) of the R25 slotted block II (30) is slidingly connected with the slot of the rear slotted block of the vertical slotted block pair IV (29) and the opened slot IV (p2) of the limiting plate II (31).

7. The bioinspired multi-axial ankle prosthesis enabling adjustable joint orientations based on fixed axis length according to claim 1, wherein the bottom cover (G) is provided with the spring seat VI (r1) located forwardly on a transverse central line in the front-and-rear direction on an upper face thereof, and the spring seat VII (r1) located rearwardly on the transverse central line in the front-and-rear direction on the upper face thereof.

## Patentansprüche

1. Bioinspirierte mehrachsige Sprunggelenkprothese, die auf einer festen Achslänge basiert und einstellbare Gelenkausrichtungen ermöglicht, wobei die bioinspirierte mehrachsige Sprunggelenkprothese, die auf einer festen Achslänge basiert und einstellbare Gelenkausrichtungen ermöglicht, aus einer linken Komponente (A), einer vorderen Komponente (B), einer oberen Abdeckung (C), einer Gelenkachsenkomponente (D), einer hinteren Komponente (E), einer rechten Komponente (F), einer Bodenabdeckung (G), einer Hilfsplatte I (1), einer Feder I (2), einer Feder II (3), einer Hilfsplatte II (4), einer Feder III (5) und einer Feder IV (6) besteht;
wobei ein unteres Ende einer linken Verbindungsplatte (7) der linken Komponente (A) mit einer oberen Fläche an einem linken Ende der Bodenabdeckung (G) fest verbunden ist, und ein unteres Ende einer rechten Verbindungsplatte (28) der rechten Komponente (F) mit einer oberen Fläche an einem rechten Ende der Bodenabdeckung (G) fest verbunden ist;
eine approximativ obere linke Seite einer hinteren Fläche einer vorderen Verbindungsplatte (10) der vorderen Komponente (B) mit einer approximativ oberen linken Seite einer vorderen Fläche einer hinteren Verbindungsplatte (27) der hinteren Komponente (E) durch die Hilfsplatte I (1) fest verbunden ist, und eine approximativ obere rechte Seite der hinteren Fläche der vorderen Verbindungsplatte (10) der vorderen Komponente (B) mit einer approximativ oberen rechten Seite der vorderen Fläche der hinteren Verbindungsplatte (27) der hinteren Komponente (E) durch die Hilfsplatte II (4) fest verbunden ist;
ein oberes Ende der vorderen Verbindungsplatte (10) der vorderen Komponente (B) mit einer unteren Fläche an einem vorderen Ende der oberen Abdeckung (C) fest verbunden ist, und ein oberes Ende der hinteren Verbindungsplatte (27) der hinteren Komponente (E) mit einer unteren Fläche an einem hinteren Ende der oberen Abdeckung (C) fest verbunden ist;
sich die Gelenkachsenkomponente (D) in einem Raum befindet, der von der linken Komponente (A), der rechten Komponente (F), der oberen Abdeckung (C), der vorderen Komponente (B), der hinteren Komponente (E), und der Bodenabdeckung (G) umschlossen ist;
ein Ring eines vorderen Bindeglieds (17) der Gelenkachsenkomponente (D) in einem Schlitz IV (q) eines R30-Schlitzblocks I (11) der vorderen Komponente (B) eingesetzt und mit einem R30-Schlitz I (q2) des R30-Schlitzblocks I (11) durch einen Bolzen fest verbunden ist; ein Ring eines hinteren Bindeglieds (15) der Gelenkachsenkomponente (D) in einem Schlitz VI (d1) eines R30-Schlitzblocks II (26) der hinteren Komponente (E) eingesetzt und mit einem R30-Schlitz II (d2) des R30-Schlitzblocks II (26) durch einen Bolzen fest verbunden ist; ein Ring eines linken Bindeglieds (18) der Gelenkachsenkomponente (D) in einem Schlitz II (g) eines R25-Schlitzblocks I (9) der linken Komponente (A) eingesetzt und mit einem R25-Schlitz I (g2) des R25-Schlitzblocks I (9) durch einen Bolzen fest verbunden ist; und ein Ring eines rechten Bindeglieds (16) der Gelenkachsenkomponente (D) in einem Schlitz VII (m1) eines R25-Schlitzblocks II (30) der rechten Komponente (F) eingesetzt und mit einem R25-Schlitz II (m2) des R25-Schlitzblocks II (30) durch einen Bolzen fest verbunden ist; ein linkes Ende der Feder I (2) mit einer rechten Seite eines Federsitzes II (h) der vorderen Verbindungsplatte (10) der vorderen Komponente (B) fest verbunden ist, und ein rechtes Ende der Feder I (2) mit einem Federsitz V (g1) der rechten Verbindungsplatte (28) der rechten Komponente (F) fest verbunden ist;
ein linkes Ende der Feder II (3) mit einem Federsitz I (a) der linken Verbindungsplatte (7) der linken Komponente (A) fest verbunden ist, und ein rechtes Ende der Feder II (3) mit einer linken Seite des Federsitzes II (h) der vorderen Verbindungsplatte II (10) der vorderen Komponente II (B) fest verbunden ist;
ein oberes Ende der Feder III (5) mit einem Federsitz IV (z) der hinteren Verbindungsplatte (27) der hinteren Komponente (E) fest verbunden ist, und ein unteres Ende der Feder III (5) mit einem Federsitz VII (s1) der Bodenabdeckung (G) fest verbunden ist; und
ein oberes Ende der Feder IV (6) mit einem Federsitz III (i) der vorderen Verbindungsplatte (10) der vorderen Komponente (B) fest verbunden ist, und ein unteres Ende der Feder IV (6) mit einem Federsitz VI (r1) der Bodenabdeckung (G) fest verbunden ist.

2. Bioinspirierte mehrachsige Sprunggelenkprothese, die auf einer festen Achslänge basiert und einstellbare Gelenkausrichtungen ermöglicht, nach Anspruch 1, wobei die linke Komponente (A) aus der linken Verbindungsplatte (7), einem vertikalen Schlitzblockpaar I (8) und dem R25-Schlitzblock I (9) besteht;
ein unterer Teil einer rechten Fläche der linken Verbindungsplatte (7) mit dem Federsitz I (a) in der Mitte versehen ist, und ein oberer Teil der rechten Fläche der linken Verbindungsplatte (7) symmetrisch mit einem geraden Schlitz II (c) und einem geraden Schlitz I (b) auf zwei Seiten vor und hinter der Mitte versehen ist;
das vertikale Schlitzblockpaar I (8) aus zwei vorderen und hinteren Schlitzblöcken mit der gleichen Struktur besteht; jeder der Schlitzblöcke mit einem Schlitz in der Vorwärts- und Rückwärtsrichtung versehen ist, und ein linkes Ende des Schlitzblocks rechteckig ist;
der R25-Schlitzblock I (9) mit einer Säule II (f) und einer Säule I (e) jeweils an vorderen und hinteren Enden versehen ist; der R25-Schlitzblock I (9) mit einem R25-Schlitz I (g2) in der Aufwärts- und Abwärtsrichtung versehen ist; der R25-Schlitzblock I (9) mit einem Schlitz II (g) in Links- und Rechtsrichtung versehen ist; der R25-Schlitzblock I (9) sich zwischen den zwei vorderen und hinteren Schlitzblöcken des vertikalen Schlitzblockpaares I (8) befindet; die Säule II (f) und die Säule I (e) des R25-Schlitzblocks I (9) mit den Schlitzen der zwei vorderen und hinteren Schlitzblöcken gleitend verbunden sind; und linke Enden der zwei Schlitzblöcke des vertikalen Schlitzblockpaares I (8) jeweils mit dem geraden Schlitz II (c) und dem geraden Schlitz I (b) der linken Verbindungsplatte (7) fest verbunden sind.

3. Bioinspirierte mehrachsige Sprunggelenkprothese, die auf einer festen Achslänge basiert und einstellbare Gelenkausrichtungen ermöglicht, nach Anspruch 1, wobei die vordere Komponente (B) aus der vorderen Verbindungsplatte (10), dem R30-Schlitzblock I (11), einem vertikalen Schlitzblockpaar II (12) und einer Begrenzungsplatte 1 (13) besteht; ein unteres Ende einer hinteren Seite der vorderen Verbindungsplatte (10) in der Mitte mit einer Querstange mit dem Federsitz II (h) und dem Federsitz III (i), ein oberer Teil der hinteren Fläche der vorderen Verbindungsplatte (10) in der Mitte mit einem Loch I (k) versehen ist, und linke und rechte Seiten des Lochs I (k) symmetrisch mit einem geraden Schlitz IV (1) und einem geraden Schlitz III (j) versehen sind;
eine Mitte eines oberen Teils einer vorderen Fläche der vorderen Verbindungsplatte (10) mit einem äußeren Sitz I (o) an der Position des Lochs I (k) fest verbunden ist; linke und rechte Enden des R30-Schlitzblocks I (11) jeweils mit einer Säule IV (s) und einer Säule III (r) versehen sind;
der R30-Schlitzblock I (11) mit dem R30-Schlitz I (q2) in Aufwärts- und Abwärtsrichtung versehen ist; der R30-Schlitzblock I (11) mit dem Schlitz IV (q) in der Vorwärts- und Rückwärtsrichtung versehen ist;
das Schlitzblockpaar II (12) aus zwei linken und rechten Schlitzblöcken mit der gleichen Struktur besteht; jeder der Schlitzblöcke mit einem Schlitz in der Links- und Rechtsrichtung versehen ist, und ein hinteres Ende des Schlitzblocks rechteckig ist;
ein vorderes Ende der Begrenzungsplatte I (13) mit einer Gewindestange I (u) versehen ist; linke und rechte Endplatten der Begrenzungsplatte 1 (13) symmetrisch mit einem offenen Schlitz I (t2) und einem offenen Schlitz II (t) versehen sind; eine Schraubenmutter auf dem äußeren Sitz I (o) gesetzt ist; die Gewindestange I (u) der Begrenzungsplatte I (13) in das Loch I (k) der vorderen Verbindungsplatte (10) und den äußeren Sitz I (o) eingesetzt und mit der Schraubenmutter auf dem äußeren Sitz I (o) fest verbunden ist;
der R30-Schlitzblock I (11) sich zwischen den linken und rechten Endplatten der Begrenzungsplatte I (13) und den zwei linken und rechten Schlitzblöcken des vertikalen Schlitzblockpaares II (12) befindet, und die Säule IV (s) des R30-Schlitzblocks I (11) mit dem Schlitz des linken Schlitzblocks des vertikalen Schlitzblockpaares II (12) und dem offenen Schlitz I (t2) der Begrenzungsplatte I (13) gleitend verbunden ist; die Säule III (r) des R30-Schlitzblocks I (11) mit dem Schlitz des rechten Schlitzblocks des vertikalen Schlitzblockpaares II (12) und dem offenen Schlitz II (t) der Begrenzungsplatte I (13) gleitend verbunden ist; und vordere Enden der zwei linken und rechten Schlitzblöcke des vertikalen Schlitzblockpaares II (12) jeweils mit dem geraden Schlitz IV (1) und dem geraden Schlitz III (j) der vorderen Verbindungsplatte (10) fest verbunden sind.

4. Bioinspirierte mehrachsige Sprunggelenkprothese, die auf einer festen Achslänge basiert und einstellbare Gelenkausrichtungen ermöglicht, nach Anspruch 1, wobei die Gelenkachsenkomponente (D) aus einer Sprunggelenkachsenbaugruppe (H), einer Subtalargelenkachsenbaugruppe (I), einem Bolzen (14), dem hinteren Bindeglied (15), dem rechten Bindeglied (16), dem vorderen Bindeglied (17) und dem linken Bindeglied (18) besteht;
wobei die Sprunggelenkachsenbaugruppe (H) aus einem rechten Rohr (19), einer Basis I (20) und einem linken Rohr (21) besteht; das rechte Rohr (19), die Basis I (20) und das linke Rohr (21) nacheinander fest verbunden sind; die Basis I (20) mit einem Durchgangsloch (v) in der Mitte versehen ist; die Subtalargelenkachsenbaugruppe (I) aus einem hinteren Rohr (22), einer Basis II (23) und einem vorderen Rohr (24) besteht; das hintere Rohr (22), die Basis II (23) und das vordere Rohr (24) nacheinander fest verbunden sind; die Basis II (23) mit einem Gewindeloch (w) in der Mitte versehen ist; das rechte Rohr (19) und das linke Rohr (21) kurze Rohre sind und das hintere Rohr (22) und das vordere Rohr (24) lange Rohre sind; die Basis I (20) der Sprunggelenkachsenbaugruppe (H) mit der Basis II (23) der Subtalargelenkachsenbaugruppe (I) durch den Bolzen (14) fest verbunden ist, und das linke Rohr (21) und das rechte Rohr (19) der Sprunggelenkachsenbaugruppe (H) eine Kreuzform mit dem hinteren Rohr (22) und dem vorderen Rohr (24) der Subtalargelenkachsenbaugruppe (I) bilden; der hintere Bindeglied (15), der vordere Bindeglied (17), der linke Bindeglied (18) und der rechte Bindeglied (16) die gleiche Struktur aufweisen, und jeweils durch die Verbindung einer Stange (x) und eines Rings (x1) gebildet sind; die Stange des hinteren Bindeglieds (15) in Spielpassung mit einem hinteren Abschnitt des hinteren Rohrs (22) der Subtalargelenkachsenbaugruppe (I) steht; die Stange des rechten Bindeglieds (16) in Spielpassung mit einem rechten Abschnitt des rechten Rohrs (19) der Sprunggelenkachsenbaugruppe (H) steht; die Stange des vorderen Bindeglieds (17) in Spielpassung mit einem vorderen Abschnitt des vorderen Rohrs (26) der Subtalargelenkachsenbaugruppe (I) steht; und die Stange des linken Bindeglieds (18) in Spielpassung mit einem linken Abschnitt des linken Rohrs (21) der Sprunggelenkachsenbaugruppe (H) steht.

5. Bioinspirierte mehrachsige Sprunggelenkprothese, die auf einer festen Achslänge basiert und einstellbare Gelenkausrichtungen ermöglicht, nach Anspruch 1, wobei die hintere Komponente (E) aus einem vertikalen Schlitzblockpaar III (25), dem R30-Schlitzblock II (26) und der hinteren Verbindungsplatte 27 besteht;
wobei das vertikale Schlitzblockpaar III (25) aus zwei linken und rechten Schlitzblöcken mit der gleichen Struktur besteht; jeder der Schlitzblöcke mit einem Schlitz in der Links- und Rechtsrichtung versehen ist, und ein hinteres Ende des Schlitzblocks rechteckig ist; der R30-Schlitzblock II (26) jeweils mit einer Säule VI (f1) und einer Säule V (e1) an linken und rechten Enden versehen ist; der R30-Schlitzblock II (30) mit dem R30-Schlitz II (d2) in der Aufwärts- und Abwärtsrichtung versehen ist; der R30-Schlitzblock II (30) mit dem Schlitz VI (dl) in der Vorwärts- und Rückwärtsrichtung versehen ist; ein unteres Ende einer vorderen Fläche der hinteren Verbindungsplatte (27) in der Mitte mit einer Querstange mit dem Federsitz IV (z) versehen ist; ein approximativ unteres Ende der vorderen Fläche der hinteren Verbindungsplatte (27) symmetrisch mit einem geraden Schlitz VI (b1) und einem geraden Schlitz V (a1) in der Links- und Rechtsrichtung versehen ist; sich der R30-Schlitzblock II (26) zwischen den zwei linken und rechten Schlitzblöcken des vertikalen Schlitzblockpaares III (25) befindet; die Säule VI (f1) und die Säule V (e1) des R30-Schlitzblocks II (26) mit den Schlitzen der zwei linken und rechten Schlitzblöcke gleitend verbunden sind; und hintere Enden der zwei linken und rechten Schlitzblöcke des vertikalen Schlitzblockpaares III (25) jeweils mit dem geraden Schlitz VI (b1) und dem geraden Schlitz V (a1) der hinteren Verbindungsplatte (27) fest verbunden sind.

6. Bioinspirierte mehrachsige Sprunggelenkprothese, die auf einer festen Achslänge basiert und einstellbare Gelenkausrichtungen ermöglicht, nach Anspruch 1, wobei die rechte Komponente (F) aus der rechten Verbindungsplatte (28), einem vertikalen Schlitzblockpaar IV (29), dem R25-Schlitzblock II (30) und einer Begrenzungsplatte II (31) besteht;
ein unterer Abschnitt der rechten Verbindungsplatte (28) mit dem Federsitz V (g1) in der Mitte versehen ist, und ein oberer Abschnitt der rechten Verbindungsplatte (28) mit einem Loch II (h1) in der Mitte versehen ist, das symmetrisch mit einem geraden Schlitz VII (i1) und einem geraden Schlitz VIII (j1) auf zwei vorderen und hinteren Seiten versehen ist; eine Mitte eines oberen Teils einer rechten Fläche der rechten Verbindungsplatte (28) mit einem äußeren Sitz II (k1) an der Position des Lochs II (h1) fest verbunden ist;
das vertikale Schlitzblockpaar IV (29) aus zwei vorderen und hinteren Schlitzblöcken mit der gleichen Struktur besteht; jeder der Schlitzblöcke mit einem Schlitz in der Vorwärts- und Rückwärtssichtung versehen ist, und ein rechtes Ende des Schlitzblocks rechteckig ist;
vordere und hintere Enden des R25-Schlitzblocks II (30) jeweils mit einer Säule VII (11) und einer Säule VIII (n1) versehen sind; der R25-Schlitzblock II (30) mit dem R25-Schlitz II (m2) in der Aufwärts- und Abwärtsrichtung versehen ist; der R25-Schlitzblock II (30) mit dem Schlitz VII (m1) in der Links- und Rechtsrichtung versehen ist;
eine rechte Fläche der Begrenzungsplatte II (31) mit einer Gewindestange II (q1) in der Mitte versehen ist; vordere und hintere Platten der Begrenzungsplatte II (31) symmetrisch mit einem offenen Schlitz III (p1) und einem offenen Schlitz IV (p2) versehen sind; eine Schraubenmutter auf dem äußeren Sitz II (k1) gesetzt ist; die Gewindestange II (q1) der Begrenzungsplatte II (31) in das Loch II (h1) der rechten Verbindungsplatte (28) und den äußeren Sitz II (k1) eingesetzt und mit der Schraubenmutter auf dem äußeren Sitz II (k1) fest verbunden ist;
rechte Enden der zwei vorderen und hinteren Schlitzblöcke des vertikalen Schlitzblockpaares IV (29) jeweils mit dem geraden Schlitz VII (i1) und dem geraden Schlitz VIII (j1) der rechten Verbindungsplatte (28) fest verbunden sind; sich der R25-Schlitzblock II (30) zwischen den vorderen und hinteren Platten der Begrenzungsplatte II (31) und den zwei vorderen und hinteren Schlitzblöcken des vertikalen Schlitzblockpaares IV (29) befindet, und die Säule III (11) des R25-Schlitzblocks II (30) mit dem Schlitz des vorderen Schlitzblocks des vertikalen Schlitzblockpaares IV (29) und dem offenen Schlitz III (p1) der Begrenzungsplatte II (31) gleitend verbunden ist; und die Säule VIII (n1) des R25-Schlitzblocks II (30) mit dem Schlitz des hinteren Schlitzblocks des vertikalen Schlitzblockpaares IV (29) und dem offenen Schlitz IV (p2) der Begrenzungsplatte II (31) gleitend verbunden ist.

7. Bioinspirierte mehrachsige Sprunggelenkprothese, die auf einer festen Achslänge basiert und einstellbare Gelenkausrichtungen ermöglicht, nach Anspruch 1, wobei die Bodenabdeckung (G) mit dem Federsitz VI (r1) versehen ist, der sich auf einer transversalen Mittellinie in der Vorwärts- und Rückwärtsrichtung auf der oberen Fläche davon nach vorne angeordnet ist, und der Federsitz VII (r1) auf der transversalen Mittellinie in der Vorwärts- und Rückwärtsrichtung auf der oberen Fläche davon nach hinten angeordnet ist.

## Revendications

1. Prothèse de cheville multi-axiale bio-inspirée permettant des orientations d'articulation réglables basées sur une longueur d'axe fixe, dans laquelle la prothèse de cheville multi-axiale bio-inspirée permettant des orientations d'articulation réglables basées sur une longueur d'axe fixe est composée d'un composant gauche (A), d'un composant avant (B), d'un couvercle supérieur (C), d'un composant d'axe d'articulation (D), d'un composant arrière (E), d'un composant droit (F), d'un couvercle inférieur (G), d'une plaque auxiliaire I (1), d'un ressort I (2), d'un ressort II (3), d'une plaque auxiliaire II (4), d'un ressort III (5) et d'un ressort IV (6);
dans laquel une extrémité inférieure d'une plaque de liaison gauche (7) du composant gauche (A) est relié fixement à une face supérieure sur une extrémité gauche du couvercle inférieur (G), et une extrémité inférieure d'une plaque de liaison droite (28) du composant droit (F) est relié fixement à une face supérieure sur une extrémité droite du couvercle inférieur (G);
un côté en haut à gauche approximativement d'une face arrière d'une plaque de liaison avant (10) du composant avant (B) est relié fixement à un côté en haut à gauche approximativement d'une face avant d'une plaque de liaison arrière (27) du composant arrière (E) par la plaque auxiliaire I (1), et un côté en haut à droit approximativement de la face arrière de la plaque de liaison avant (10) du composant avant (B) est relié fixement à un côté en haut à droit approximativement de la face avant de la plaque de liaison arrière (27) du composant arrière (E) par la plaque auxiliaire II (4);
une extrémité supérieure de la plaque de liaison avant (10) du composant avant (B) est reliée fixement à une face inférieure sur une extrémité avant du couvercle supérieur (C), et une extrémité supérieure de la plaque de liaison arrière (27) du composant arrière (E) est relié fixement à une face inférieure sur une extrémité arrière du couvercle supérieur (C);
le composant d'axe d'articulation (D) est localisé dans un espace entouré par le composant gauche (A), le composant droit (F), le couvercle supérieur (C), le composant avant (B), le composant arrière (E), et le couvercle inférieur (G);
un anneau d'une bielle avant (17) du composant d'axe d'articulation (D) est inséré dans une fente IV (q) d'un bloc fendu R30 I (11) du composant avant (B), et est relié fixement à un R30 fente I (q2) du bloc fendu R30 I (11) par un boulon ; un anneau d'une bielle arrière (15) du composant d'axe d'articulation (D) est inséré dans une fente VI (dl) d'un bloc fendu R30 II (26) du composant arrière (E), et est relié fixement à un R30 fente II (d2) du bloc fendu R30 II (26) par un boulon ; un anneau d'une bielle gauche (18) du composant d'axe d'articulation (D) est inséré dans une fente II (g) d'un bloc fendu R25 I (9) du composant gauche (A), et est relié fixement à un R25 fente I (g2) du bloc fendu R25 I (9) par un boulon ; et un anneau d'une bielle droite (16) du composant d'axe d'articulation (D) est inséré dans une fente VII (ml) d'un bloc fendu R25 II (30) du composant droit (F), et est relié fixement à une fente R25 II (m2) du bloc fendu R25 II (30) par un boulon;
une extrémité gauche du ressort I (2) est reliée fixement à un côté droit d'un siège de ressort II (h) de la plaque de liaison avant (10) du composant avant (B), et une extrémité droite du ressort I (2) est reliée fixement à un siège de ressort V (gl) de la plaque de liaison droite (28) du composant droit (F);
une extrémité gauche du ressort II (3) est reliée fixement à un siège de ressort I (a) de la plaque de liaison gauche (7) du composant gauche (A), et une extrémité droite du ressort II (3) est reliée fixement à un côté gauche du siège de ressort II (h) de la plaque de liaison avant II (10) du composant avant II (B);
une extrémité supérieure du ressort III (5) est reliée fixement à un siège de ressort IV (z) de la plaque de liaison arrière (27) du composant arrière (E), et une extrémité inférieure du ressort III (5) est reliée fixement à un siège de ressort VII (s1) du couvercle inférieur (G); et
une extrémité supérieure du ressort IV (6) est reliée fixement à un siège de ressort III (i) de la plaque de liaison avant (10) du composant avant (B), et une extrémité inférieure du ressort IV (6) est reliée fixement à un siège de ressort VI (rl) du couvercle inférieur (G).

2. Prothèse de cheville multi-axiale bio-inspirée permettant des orientations d'articulation réglables basées sur une longueur d'axe fixe selon la revendication 1, dans laquelle le composant gauche (A) est composé de la plaque de liaison gauche (7), d'une paire de blocs fendus verticaux I (8) et du bloc fendu R25 I (9);
une partie inférieure d'une face droite de la plaque de liaison gauche (7) est pourvue du siège de ressort I (a) au centre, et une partie supérieure de la face droite de la plaque de liaison gauche (6) est pourvue symétriquement d'une fente droite II (c) et une fente droite I (b) sur deux côtés devant et derrière le centre;
la paire de blocs fendus verticaux I (8) est composée de deux blocs fendus avant et arrière ayant la même structure; chacun des blocs fendus est pourvu d'une fente dans le sens avant et arrière, et une extrémité gauche du bloc fendus est rectangulaire;
le bloc fendu R25 I (9) est pourvu d'une colonne II (f) et d'une colonne I (e) sur les extrémités avant et arrière respectivement ; le bloc fendu R25 I (9) est pourvu d'une fente R25 I (g2) dans le sens haut-et-bas; le bloc fendus R25 I (9) est pourvu d'une fente II (g) dans le sens gauche-et-droit; le bloc fendu R25 I (9) est localisé entre les deux blocs fendus avant et arrière de la paire de blocs fendus verticaux I (8); la colonne II (f) et la colonne I (e) du bloc fendu R25 I (9) sont reliées de manière coulissantes aux fentes des deux blocs fendus avant et arrière; et les extrémités gauches des deux blocs fendus de la paire de blocs fendus verticaux I (8) sont reliées fixement à la fente droite II (c) et la fente droite I (b) de la plaque de liaison gauche (7) respectivement.

3. Prothèse de cheville multi-axiale bio-inspirée permettant des orientations d'articulation réglables basées sur une longueur d'axe fixe selon la revendication 1, dans laquelle le composant avant (B) est composé de la plaque de liaison avant (10), du bloc fendu R30 I (11), d'une paire de blocs fendus verticaux II (12) et d'une plaque de limitation 1 (13);
une extrémité inférieure d'une face arrière de la plaque de liaison avant (10) est pourvue au centre d'une barre transversale avec le siège de ressort II (h) et le siège de ressort III (i), une partie supérieure de la face arrière de la plaque avant la plaque de liaison (10) est pourvue au centre d'un trou I (k), et les côtés gauche et droit du trou I (k) sont pourvus symétriquement d'une fente droite IV (1) et d'une fente droite III (j);
un centre d'une partie supérieure d'une face avant de la plaque de liaison avant (10) est relié fixement à un siège extérieur I (o) à la position du trou I (k); les extrémités gauche et droite du bloc fendu R30 I (11) sont pourvues d'une colonne IV(s) et d'une colonne III(r) respectivement;
le bloc fendu R30 I (11) est pourvu de la fente R30 I (q2) dans le sens haut-et-bas; le bloc fendu R30 I (11) est pourvu de la fente IV (q) dans le sens avant-et-arrière;
la paire de blocs fendus verticaux II (12) est composée de deux blocs fendus gauche et droit ayant la même structure; chacun des blocs fendus est pourvu d'une fente dans le sens gauche-et-droit, et une extrémité arrière du bloc fendu est rectangulaire;
une extrémité avant de la plaque de limitation I (13) est pourvue d'une tige filetée I (u); les plaques d'extrémité gauche et droite de la plaque de limitation 1 (13) sont pourvues symétriquement d'une fente ouverte I (t2) et d'une fente ouverte II (t); un écrou est placé sur le siège extérieur I (o); la tige filetée I (u) de la plaque de limitation I (13) est insérée dans le trou I (k) de la plaque de liaison avant (10) et du siège extérieur I (o), et est reliée fixement à l'écrou sur le siège extérieur I (o);
le bloc fendu R30 I (11) est localisé entre les plaques d'extrémité gauche et droite de la plaque de limitation I (13) et les deux blocs fendus gauche et droit de la paire de blocs fendus verticaux II (12), et la colonne IV (s) du bloc fendu R30 I (11) est relié de manière coulissante à la fente du bloc fendu gauche de la paire de blocs fendus verticaux II (12) et à la fente ouverte I (t2) de la plaque de limitation I (13); la colonne III (r) du bloc fendu R30 I (11) est reliée de manière coulissante à la fente du bloc fendu droit de la paire de blocs fendus verticaux II (12) et à la fente ouverte II (t) de la plaque de limitation I (13); et les extrémités avant des deux blocs fendus gauche et droit de la paire de blocs fendus verticaux II (12) sont reliées fixement à la fente droite IV (1) et à la fente droite III (j) de la plaque de liaison avant (10) respectivement.

4. Prothèse de cheville multi-axiale bio-inspirée permettant des orientations d'articulation réglables basées sur une longueur d'axe fixe selon la revendication 1, dans laquelle le composant d'axe articulaire (D) est composé d'un ensemble d'axe articulaire de cheville (H), d'un ensemble d'axe articulaire sous-astragalien (I), d'un boulon (14), de la bielle arrière (15), de la bielle droite (16), de la bielle avant (17) et la bielle gauche (18);
dans laquelle l'ensemble d'axe d'articulation de cheville (H) est composé d'un tube droit (19), d'une base I (20) et d'un tube gauche (21); le tube droit (19), la base I (20) et le tube gauche (21) sont reliés fixement en séquence; la base I (20) est pourvue d'un trou traversant (v) au centre; l'ensemble d'axe articulaire sous-astragalien (I) est composé d'un tube arrière (22), d'une base II (23) et d'un tube avant (24); le tube arrière (22), la base II (23) et le tube avant (24) sont reliés fixement en séquence; la base II (23) est pourvue d'un trou fileté (w) au centre; le tube droit (19) et le tube gauche (21) sont des tubes courts, et le tube arrière (22) et le tube avant (24) sont des tubes longs ; la base I (20) de l'ensemble d'axe d'articulation de cheville (H) est reliée fixement à la base II (23) de l'ensemble d'axe d'articulation sous-astragalien (I) par le boulon (14), et le tube gauche (21) et le tube droit (19) de l'ensemble d'axe d'articulation de cheville (H) forme une croix avec le tube arrière (22) et le tube avant (24) de l'ensemble d'axe d'articulation sous-astragalien (I); la bielle arrière (15), la bielle avant (17), la bielle gauche (18) et la bielle droite (16) ont la même structure, et elles sont formées chacune par reliant une tige (x) à un anneau (xl); la tige de la bielle arrière (15) est adaptée avec jeu à une partie arrière du tube arrière (22) de l'ensemble d'axe d'articulation sous-astragalien (I); la tige de la bielle droite (16) est adaptée avec jeu à une partie droite du tube droit (19) de l'ensemble d'axe d'articulation de cheville (H); la tige de la bielle avant (17) est adaptée avec jeu à une partie avant du tube avant (26) de l'ensemble d'axe d'articulation sous-astragalien (I); et la tige de la bielle gauche (18) est adaptée avec jeu à une partie gauche du tube gauche (21) de l'ensemble d'axe d'articulation de cheville (H).

5. Prothèse de cheville multi-axiale bio-inspirée permettant des orientations d'articulation réglables basées sur une longueur d'axe fixe selon la revendication 1, dans laquelle le composant arrière (E) est composé d'une paire de blocs fendus verticaux III (25), du bloc fendu R30 II (26), et de la plaque de liaison arrière 27;
dans laquelle la paire de blocs fendus verticaux III (25) est composée de deux blocs fendus gauche et droit ayant la même structure; chacun des blocs fendus est pourvu d'une fente dans le sens gauche-et-droit, et une extrémité arrière du bloc fendu est rectangulaire;
le bloc fendu R30 II (26) est pourvu d'une colonne VI (f1) et d'une colonne V (el) aux extrémités gauche et droite respectivement; le bloc fendu R30 II (30) est pourvu de la fente R30 II (d2) dans le sens haut-et-bas; le bloc fendu R30 II (30) est pourvu de la fente VI (dl) dans le sens avant-et-arrière; une extrémité inférieure d'une face avant de la plaque de liaison arrière (27) est pourvue au centre d'une barre transversale avec le siège de ressort IV (z); une extrémité approximativement inférieure de la face avant de la plaque de liaison arrière (27) est pourvue de manière symétrique d'une fente droite VI (bl) et d'une fente droite V (al) dans le sens gauche-et-droit; le bloc fendu R30 II (26) est localisé entre les deux blocs fendus gauche et droit de la paire de blocs fendus verticaux III (25); la colonne VI (fl) et la colonne V (el) du bloc fendu R30 II (26) sont reliées de manière coulissante aux fentes des deux blocs fendus gauche et droit; et les extrémités arrière des deux blocs fendus gauche et droit de la paire de blocs fendus verticaux III (25) sont reliées fixement à la fente droite VI (bl) et la fente droite V (al) de la plaque de liaison arrière (27) respectivement.

6. Prothèse de cheville multi-axiale bio-inspirée permettant des orientations d'articulation réglables basées sur une longueur d'axe fixe selon la revendication 1, dans laquelle le composant droit (F) est composé de la plaque de liaison droite (28), d'une paire de blocs fendus verticaux IV (29) , du bloc fendu R25 II (30) et d'une plaque de limitation II (31);
une partie inférieure de la plaque de liaison droite (28) est pourvue du siège de ressort V (gl) au centre, et une partie supérieure de la plaque de liaison droite (28) est pourvue d'un trou II (h1) au centre, qui est pourvue symétriquement d'une fente droite VII (il) et d'une fente droite VIII (j1) sur deux côtés avant et arrière; un centre d'une partie supérieure d'une face droite de la plaque de liaison droite (28) est relié fixement à un siège extérieur II (kl) à la position du trou II (h1);
la paire de blocs fendus verticaux IV (29) est composée de deux blocs fendus avant et arrière ayant la même structure; chacun des blocs fendus est pourvu d'une fente dans le sens avant-et-arrière, et une extrémité droite du bloc fendu est rectangulaire;
les extrémités avant et arrière du bloc fendu R25 II (30) sont pourvues d'une colonne VII (11) et d'une colonne VIII (n1) respectivement; le bloc fendu R25 II (30) est pourvu de la fente R25 II (m2) dans le sens haut-et-bas; le bloc fendu R25 II (30) est pourvu de la fente VII (m1) dans le sens gauche-et-droit;
une face droite de la plaque de limitation II (31) est pourvue d'une tige filetée II (ql) au centre; des plaques avant et arrière de la plaque de limitation II (31) sont pourvues symétriquement d'une fente ouverte III (p1) et d'une fente ouverte IV (p2); un écrou est placé sur le siège extérieur II (kl); la tige filetée II (ql) de la plaque de limitation II (31) est insérée dans le trou II (h1) de la plaque de liaison droite (28) et du siège extérieur II (kl), et est reliée fixement à l'écrou sur le siège extérieur II (kl);
des extrémités droites des deux blocs fendus avant et arrière de la paire de blocs fendus verticaux IV (29) sont reliées fixement à la fente droite VII (il) et à la fente droite VIII (jl) de la plaque de liaison droite (28) respectivement; le bloc fendu R25 II (30) est localisé entre les plaques avant et arrière de la plaque de limitation II (31) et les deux blocs fendus avant et arrière de la paire de blocs fendus verticaux IV (29), et la colonne III (11) du bloc fendu R25 II (30) est reliée de manière coulissante à la fente du bloc fendu avant de la paire de blocs fendus verticaux IV (29) et à la fente ouverte III (p1) de la plaque de limitation II (31); et la colonne VIII (nl) du bloc fendu R25 II (30) est reliée de manière coulissante à la fente du bloc fendu arrière de la paire de blocs fendus verticaux IV (29) et à la fente ouverte IV (p2) de la plaque de limitation II (31).

7. Prothèse de cheville multi-axiale bio-inspirée permettant des orientations d'articulation réglables basées sur une longueur d'axe fixe selon la revendication 1, dans laquelle le couvercle inférieur (G) est pourvu du siège de ressort VI (rl) localisé devant d'une ligne médiane transversale dans le sens avant-et-arrière sur sa face supérieure, et le siège de ressort VII (rl) localisé derrière de la ligne médiane transversale dans le sens avant-et-arrière sur sa face supérieure.
